# EUROPEAN PATENT APPLICATION

(11) **EP 3 333 179 A1**
(43) Date of publication of application: **13.06.2018**
(21) Application number: 16202580.3
(22) Date of filing: 07.12.2016
(51) Int. Cl.: C07K 14/33

(54) **NOVEL RECOMBINANT BOTULINUM TOXIN WITH ACCELARATED ONSET OF EFFECT**

(71) Applicant: Merz Pharma GmbH & Co. KGaA, 60318 Frankfurt am Main (DE)
(72) Inventor: Frevert, Jürgen, 10625 Berlin (DE); Hofmann, Fred, 14480 Potsdam (DE); López de la Paz, Manuela, 65835 Liederbach am Taunus (DE); Scheps, Daniel, 14471 Potsdam (DE)
(74) Representative: Hackenberg, Julia

(57) **Abstract**

This invention relates to novel recombinant clostridial neurotoxins exhibiting an accelerated onset of effect and to methods for the manufacture of such recombinant clostridial neurotoxins. These novel recombinant clostridial neurotoxins comprise a domain consisting of proline, glycine and serine residues, and the methods comprise the steps of inserting a nucleic acid sequence coding for said domain into a nucleic acid sequence coding for a parental clostridial neurotoxin and expression of the recombinant nucleic acid sequence comprising said domain-coding sequence in a host cell. The invention further relates to novel recombinant single-chain precursor clostridial neurotoxins used in such methods, nucleic acid sequences encoding such recombinant single-chain precursor clostridial neurotoxins, and pharmaceutical compositions comprising the recombinant clostridial neurotoxin with an accelerated onset of effect.

SEQUENCE LISTING

<110> Merz Pharma GmbH & Co. KGaA

<120> NOVEL RECOMBINANT BOTULINUM TOXIN WITH ACCELARATED ONSET OF EFFECT

<130> 4115-000-EP

<160> 3

<170> BiSSAP 1.3.5

<210> 1
<211> 20
<212> PRT
<213> Artificial Sequence

<223> "PGS"

<220>
<223> PGS

<400> 1
Gly Ser Pro Gly Gly Pro Gly Pro Gly Ser Pro Gly Gly Pro Gly Pro
1               5                   10                  15
Ser Gly Pro Gly
            20

<210> 2
<211> 1446
<212> PRT
<213> Artificial Sequence

<223> "PGS-100-BoNT/A "

<220>
<223> PGS-100-BoNT/A

<400> 2
   Met Gly Ser Ser His His His His His His Gly Ser Leu Val Pro Arg
   1               5                   10                  15
   Ser Ser Ser Gly Ser Pro Gly Gly Pro Gly Pro Gly Ser Pro Gly Gly
               20                  25                  30
   Pro Gly Pro Ser Gly Pro Gly Gly Ser Pro Gly Gly Pro Gly Pro Gly
           35                  40                  45
   Ser Pro Gly Gly Pro Gly Pro Ser Gly Pro Gly Gly Ser Pro Gly Gly
       50                  55                  60
   Pro Gly Pro Gly Ser Pro Gly Gly Pro Gly Pro Ser Gly Pro Gly Gly
   65                  70                  75                  80
   Ser Pro Gly Gly Pro Gly Pro Gly Ser Pro Gly Gly Pro Gly Pro Ser
                   85                  90                  95
   Gly Pro Gly Gly Ser Pro Gly Gly Pro Gly Pro Gly Ser Pro Gly Gly
               100                 105                 110
   Pro Gly Pro Ser Gly Pro Gly Gly Pro Phe Val Asn Lys Gln Phe Asn
           115                 120                 125
   Tyr Lys Asp Pro Val Asn Gly Val Asp Ile Ala Tyr Ile Lys Ile Pro
       130                 135                 140
   Asn Ala Gly Gln Met Gln Pro Val Lys Ala Phe Lys Ile His Asn Lys
   145                 150                 155                 160
   Ile Trp Val Ile Pro Glu Arg Asp Thr Phe Thr Asn Pro Glu Glu Gly
                   165                 170                 175
   Asp Leu Asn Pro Pro Pro Glu Ala Lys Gln Val Pro Val Ser Tyr Tyr
               180                 185                 190
   Asp Ser Thr Tyr Leu Ser Thr Asp Asn Glu Lys Asp Asn Tyr Leu Lys
           195                 200                 205
   Gly Val Thr Lys Leu Phe Glu Arg Ile Tyr Ser Thr Asp Leu Gly Arg
       210                 215                 220
   Met Leu Leu Thr Ser Ile Val Arg Gly Ile Pro Phe Trp Gly Gly Ser
   225                 230                 235                 240
   Thr Ile Asp Thr Glu Leu Lys Val Ile Asp Thr Asn Cys Ile Asn Val
                   245                 250                 255
   Ile Gln Pro Asp Gly Ser Tyr Arg Ser Glu Glu Leu Asn Leu Val Ile
               260                 265                 270
   Ile Gly Pro Ser Ala Asp Ile Ile Gln Phe Glu Cys Lys Ser Phe Gly
           275                 280                 285
   His Glu Val Leu Asn Leu Thr Arg Asn Gly Tyr Gly Ser Thr Gln Tyr
       290                 295                 300
   Ile Arg Phe Ser Pro Asp Phe Thr Phe Gly Phe Glu Glu Ser Leu Glu
   305                 310                 315                 320
   Val Asp Thr Asn Pro Leu Leu Gly Ala Gly Lys Phe Ala Thr Asp Pro
                   325                 330                 335
   Ala Val Thr Leu Ala His Glu Leu Ile His Ala Gly His Arg Leu Tyr
               340                 345                 350
   Gly Ile Ala Ile Asn Pro Asn Arg Val Phe Lys Val Asn Thr Asn Ala
           355                 360                 365
   Tyr Tyr Glu Met Ser Gly Leu Glu Val Ser Phe Glu Glu Leu Arg Thr
       370                 375                 380
   Phe Gly Gly His Asp Ala Lys Phe Ile Asp Ser Leu Gln Glu Asn Glu
   385                 390                 395                 400
   Phe Arg Leu Tyr Tyr Tyr Asn Lys Phe Lys Asp Ile Ala Ser Thr Leu
                   405                 410                 415
   Asn Lys Ala Lys Ser Ile Val Gly Thr Thr Ala Ser Leu Gln Tyr Met
               420                 425                 430
   Lys Asn Val Phe Lys Glu Lys Tyr Leu Leu Ser Glu Asp Thr Ser Gly
           435                 440                 445
   Lys Phe Ser Val Asp Lys Leu Lys Phe Asp Lys Leu Tyr Lys Met Leu
       450                 455                 460
   Thr Glu Ile Tyr Thr Glu Asp Asn Phe Val Lys Phe Phe Lys Val Leu
   465                 470                 475                 480
   Asn Arg Lys Thr Tyr Leu Asn Phe Asp Lys Ala Val Phe Lys Ile Asn
                   485                 490                 495
   Ile Val Pro Lys Val Asn Tyr Thr Ile Tyr Asp Gly Phe Asn Leu Arg
               500                 505                 510
   Asn Thr Asn Leu Ala Ala Asn Phe Asn Gly Gln Asn Thr Glu Ile Asn
           515                 520                 525
   Asn Met Asn Phe Thr Lys Leu Lys Asn Phe Thr Gly Leu Phe Glu Phe
       530                 535                 540
   Tyr Lys Leu Leu Cys Val Arg Gly Ile Ile Thr Ser Lys Ala Gly Ala
   545                 550                 555                 560
   Gly Lys Ser Leu Val Pro Arg Gly Ser Ala Gly Ala Gly Ala Leu Asn
                   565                 570                 575
   Asp Leu Cys Ile Lys Val Asn Asn Trp Asp Leu Phe Phe Ser Pro Ser
               580                 585                 590
   Glu Asp Asn Phe Thr Asn Asp Leu Asn Lys Gly Glu Glu Ile Thr Ser
           595                 600                 605
   Asp Thr Asn Ile Glu Ala Ala Glu Glu Asn Ile Ser Leu Asp Leu Ile
       610                 615                 620
   Gln Gln Tyr Tyr Leu Thr Phe Asn Phe Asp Asn Glu Pro Glu Asn Ile
   625                 630                 635                 640
   Ser Ile Glu Asn Leu Ser Ser Asp Ile Ile Gly Gln Leu Glu Leu Met
                   645                 650                 655
   Pro Asn Ile Glu Arg Phe Pro Asn Gly Lys Lys Tyr Glu Leu Asp Lys
               660                 665                 670
   Tyr Thr Met Phe His Tyr Leu Arg Ala Gln Glu Phe Glu His Gly Lys
           675                 680                 685
   Ser Arg Ile Ala Leu Thr Asn Ser Val Asn Glu Ala Leu Leu Asn Pro
   690                 695                 700
   Ser Arg Val Tyr Thr Phe Phe Ser Ser Asp Tyr Val Lys Lys Val Asn
   705                 710                 715                 720
   Lys Ala Thr Glu Ala Ala Met Phe Leu Gly Trp Val Glu Gln Leu Val
                   725                 730                 735
   Tyr Asp Phe Thr Asp Glu Thr Ser Glu Val Ser Thr Thr Asp Lys Ile
               740                 745                 750
   Ala Asp Ile Thr Ile Ile Ile Pro Tyr Ile Gly Pro Ala Leu Asn Ile
           755                 760                 765
   Gly Asn Met Leu Tyr Lys Asp Asp Phe Val Gly Ala Leu Ile Phe Ser
       770                 775                 780
   Gly Ala Val Ile Leu Leu Glu Phe Ile Pro Glu Ile Ala Ile Pro Val
   785                 790                 795                 800
   Leu Gly Thr Phe Ala Leu Val Ser Tyr Ile Ala Asn Lys Val Leu Thr
                   805                 810                 815
   Val Gln Thr Ile Asp Asn Ala Leu Ser Lys Arg Asn Glu Lys Trp Asp
               820                 825                 830
   Glu Val Tyr Lys Tyr Ile Val Thr Asn Trp Leu Ala Lys Val Asn Thr
           835                 840                 845
   Gln Ile Asp Leu Ile Arg Lys Lys Met Lys Glu Ala Leu Glu Asn Gln
       850                 855                 860
   Ala Glu Ala Thr Lys Ala Ile Ile Asn Tyr Gln Tyr Asn Gln Tyr Thr
   865                 870                 875                 880
   Glu Glu Glu Lys Asn Asn Ile Asn Phe Asn Ile Asp Asp Leu Ser Ser
                   885                 890                 895
   Lys Leu Asn Glu Ser Ile Asn Lys Ala Met Ile Asn Ile Asn Lys Phe
               900                 905                 910
   Leu Asn Gln Cys Ser Val Ser Tyr Leu Met Asn Ser Met Ile Pro Tyr
           915                 920                 925
   Gly Val Lys Arg Leu Glu Asp Phe Asp Ala Ser Leu Lys Asp Ala Leu
       930                 935                 940
   Leu Lys Tyr Ile Tyr Asp Asn Arg Gly Thr Leu Ile Gly Gln Val Asp
   945                 950                 955                 960
   Arg Leu Lys Asp Lys Val Asn Asn Thr Leu Ser Thr Asp Ile Pro Phe
                   965                 970                 975
   Gln Leu Ser Lys Tyr Val Asp Asn Gln Arg Leu Leu Ser Thr Phe Thr
               980                 985                 990
   Glu Tyr Ile Lys Asn Ile Ile Asn Thr Ser Ile Leu Asn Leu Arg Tyr
           995                 1000                1005
   Glu Ser Asn His Leu Ile Asp Leu Ser Arg Tyr Ala Ser Lys Ile Asn
       1010                1015                1020
   Ile Gly Ser Lys Val Asn Phe Asp Pro Ile Asp Lys Asn Gln Ile Gln
   1025                1030                1035                1040
   Leu Phe Asn Leu Glu Ser Ser Lys Ile Glu Val Ile Leu Lys Asn Ala
                   1045                1050                1055
   Ile Val Tyr Asn Ser Met Tyr Glu Asn Phe Ser Thr Ser Phe Trp Ile
               1060                1065                1070
   Arg Ile Pro Lys Tyr Phe Asn Ser Ile Ser Leu Asn Asn Glu Tyr Thr
           1075                1080                1085
   Ile Ile Asn Cys Met Glu Asn Asn Ser Gly Trp Lys Val Ser Leu Asn
       1090                1095                1100
   Tyr Gly Glu Ile Ile Trp Thr Leu Gln Asp Thr Gln Glu Ile Lys Gln
   1105                1110                1115                1120
   Arg Val Val Phe Lys Tyr Ser Gln Met Ile Asn Ile Ser Asp Tyr Ile
                   1125                1130                1135
   Asn Arg Trp Ile Phe Val Thr Ile Thr Asn Asn Arg Leu Asn Asn Ser
               1140                1145                1150
   Lys Ile Tyr Ile Asn Gly Arg Leu Ile Asp Gln Lys Pro Ile Ser Asn
           1155                1160                1165
   Leu Gly Asn Ile His Ala Ser Asn Asn Ile Met Phe Lys Leu Asp Gly
       1170                1175                1180
   Cys Arg Asp Thr His Arg Tyr Ile Trp Ile Lys Tyr Phe Asn Leu Phe
   1185                1190                1195                1200
   Asp Lys Glu Leu Asn Glu Lys Glu Ile Lys Asp Leu Tyr Asp Asn Gln
                   1205                1210                1215
   Ser Asn Ser Gly Ile Leu Lys Asp Phe Trp Gly Asp Tyr Leu Gln Tyr
               1220                1225                1230
   Asp Lys Pro Tyr Tyr Met Leu Asn Leu Tyr Asp Pro Asn Lys Tyr Val
           1235                1240                1245
   Asp Val Asn Asn Val Gly Ile Arg Gly Tyr Met Tyr Leu Lys Gly Pro
       1250                1255                1260
   Arg Gly Ser Val Met Thr Thr Asn Ile Tyr Leu Asn Ser Ser Leu Tyr
   1265                1270                1275                1280
   Arg Gly Thr Lys Phe Ile Ile Lys Lys Tyr Ala Ser Gly Asn Lys Asp
                   1285                1290                1295
   Asn Ile Val Arg Asn Asn Asp Arg Val Tyr Ile Asn Val Val Val Lys
               1300                1305                1310
   Asn Lys Glu Tyr Arg Leu Ala Thr Asn Ala Ser Gln Ala Gly Val Glu
           1315                1320                1325
   Lys Ile Leu Ser Ala Leu Glu Ile Pro Asp Val Gly Asn Leu Ser Gln
       1330                1335                1340
   Val Val Val Met Lys Ser Lys Asn Asp Gln Gly Ile Thr Asn Lys Cys
   1345                1350                1355                1360
   Lys Met Asn Leu Gln Asp Asn Asn Gly Asn Asp Ile Gly Phe Ile Gly
                   1365                1370                1375
   Phe His Gln Phe Asn Asn Ile Ala Lys Leu Val Ala Ser Asn Trp Tyr
               1380                1385                1390
   Asn Arg Gln Ile Glu Arg Ser Ser Arg Thr Leu Gly Cys Ser Trp Glu
           1395                1400                1405
   Phe Ile Pro Val Asp Asp Gly Trp Gly Glu Arg Pro Leu Gly Asp Leu
       1410                1415                1420
   Val Pro Arg Gly Ser Ala Asn Ser Ser Ser Val Asp Lys Leu Trp Ser
   1425                1430                1435                1440
   His Pro Gln Phe Glu Lys
                   1445

<210> 3
<211> 4341
<212> DNA
<213> Artificial Sequence

<223> "PGS-100-BoNT/A  "

<220>
<223> PGS-100-BoNT/A

<400> 3
atgggtagca gccatcatca tcaccatcat ggtagcctgg ttccgcgtag cagcagcggt      60

agtccgggtg gtccgggtcc gggttcacct ggtggtcctg gaccgagcgg tcctggtggt     120

tctccaggtg gccctggtcc gggaagccca ggtggaccag gtcctagtgg tccaggcggt     180

tctcctggcg gtccaggtcc tggatctcca ggcggaccgg gtccgtcagg tccaggtggt     240

agccctggtg gcccaggccc tggctcaccg ggtggacctg gcccttcagg accgggtggg     300

tcacctgggg gaccgggacc tggaagtcct gggggtcctg gtccatcagg cccaggcggt     360

ccatttgtga acaagcagtt taactataag gacccggtga acggtgtgga tatcgcgtat     420

atcaaaatcc cgaatgcggg ccagatgcaa ccagtcaagg cgttcaagat tcataacaag     480

atttgggtta ttccggaacg tgataccttc accaatccgg aagaaggcga tttaaatccg     540

ccgccagaag ccaaacaagt gccggtgagc tactatgata gcacgtatct tagcaccgat     600

aatgaaaaag acaattacct gaagggcgtg accaagttgt tcgagcgcat ctacagtacc 660

gacttaggcc gcatgttgtt gacgagcatc gttcgcggta tcccgttctg gggcggctcg 720

accattgata ccgagttgaa agtcattgac acgaactgta tcaatgttat ccaaccggac 780

ggcagttatc gcagcgagga gttaaatttg gtcatcatcg gtccaagcgc agatattatt 840

cagttcgaat gcaagagctt cggccatgag gtcttgaatt tgacgcgcaa cggttacggc 900

agcacccaat acatccgctt tagcccggat ttcacctttg gcttcgagga gagcttggag 960

gtggacacca acccgctgtt aggtgccggc aaattcgcaa ccgacccggc agtgacgttg 1020

gcgcacgaat tgattcatgc gggtcaccgc ttatacggta tcgcgatcaa tccgaatcgc 1080

gtctttaaag tcaataccaa cgcgtactac gaaatgagcg gcttagaggt tagctttgaa 1140

gaattacgca ccttcggtgg ccacgacgcc aagttcatcg acagcctgca ggaaaatgag 1200

ttccgcttgt actattacaa taaattcaag gacatcgcga gcaccttaaa taaagcaaag 1260

agcattgtgg gcaccaccgc aagcttgcag tacatgaaga acgtatttaa ggaaaaatat 1320

ttgttgtcgg aggataccag cgggaaattc agcgtcgata agctgaaatt cgacaaattg 1380

tataaaatgc tgaccgagat ttacaccgag gataacttcg tcaagttttt taaggtgtta 1440

aatcgtaaga cctatttaaa ctttgataaa gcggtgttta aaattaatat cgtgccgaag 1500

gtgaattaca ccatctacga tggtttcaat ttacgcaaca cgaatctggc ggcgaatttt 1560

aatggccaaa acaccgaaat taacaacatg aactttacga agttaaagaa tttcacgggc 1620

ttattcgaat tctacaagtt attatgcgtg cgcggcatca ttaccagcaa ggcaggtgcg 1680

ggcaagtcct tggttccgcg tggcagcgcc ggcgccggcg cgctcaatga tctgtgtatt 1740

aaagtcaata actgggacct gttcttcagc ccgagcgagg ataactttac caacgactta 1800

aacaaaggcg aggagatcac gagcgatacg aacatcgagg cggcggagga aaatattagc 1860

ctggacctca ttcagcagta ctatctgacg ttcaattttg acaatgagcc ggagaacatc 1920

agcattgaaa atctcagcag cgacatcatc ggtcagttgg aactgatgcc gaacattgaa 1980

cgctttccga acggcaaaaa atatgaactg gacaagtata ccatgttcca ttacttacgc 2040

gcacaggaat ttgagcacgg caagagccgc attgcgctga ccaatagcgt taacgaggcc 2100

ttgttaaatc cgagccgtgt ctacacgttc ttcagcagcg attatgtcaa aaaagtgaac 2160

aaggcgaccg aagccgcgat gtttttgggc tgggtcgagc aattggttta cgattttacc 2220

gacgaaacca gcgaggtgag cacgaccgac aaaattgcag atatcaccat catcattccg 2280

tacatcggtc cggcgctcaa tatcggcaat atgttataca aggacgactt tgtgggcgcg 2340

ctgatcttta gcggcgcggt tatcttatta gaattcatcc cggagatcgc aatcccggtc 2400

ttgggcacct ttgcgttggt gagctatatc gcgaataaag tgctcacggt ccaaaccatc 2460

gataacgcgc tcagcaagcg taatgagaaa tgggacgagg tttataagta tatcgtgacc 2520

aactggttag caaaagtcaa tacgcagatc gatctcatcc gcaaaaaaat gaaagaagcc 2580

ttggaaaatc aagcggaggc aaccaaagcc atcattaatt accagtataa ccaatatacc 2640

gaagaagaaa aaaacaatat caacttcaat atcgatgatt tgagcagcaa actgaacgag 2700

agcattaaca aagcgatgat taacatcaac aagttcttga atcaatgcag cgtgagctat 2760

ctcatgaaca gcatgatccc gtatggcgtc aaacgcttgg aagattttga cgccagcctg 2820

aaagatgcgc tcctcaagta tatttatgac aaccgcggca ccctcattgg ccaggtggac 2880

cgcttgaagg ataaagtgaa caatacgctc agcacggata tcccgttcca gctgagcaag 2940

tacgtcgaca accagcgctt actgagcacc tttaccgagt atatcaagaa catcattaat 3000

accagcatcc tcaacttgcg ctatgagagc aatcacctga tcgacctcag ccgctacgcc 3060

agcaagatca acatcggcag caaggtcaat ttcgacccga tcgataagaa tcagatccaa 3120

ttgtttaacc tggaaagcag caagatcgag gttatcttga agaacgcgat tgtgtacaac 3180

agcatgtacg agaactttag cacgagcttc tggattcgta tcccgaagta tttcaatagc 3240

attagcctga ataacgaata taccattatc aactgcatgg aaaataatag cggctggaag 3300

gtgagcttaa attacggcga gatcatttgg accttacagg atacccaaga aatcaaacag 3360

cgcgtcgtct ttaagtatag ccagatgatc aacatcagcg attacatcaa ccgctggatc 3420

ttcgtgacca tcaccaataa tcgcttgaat aatagcaaga tttacatcaa tggtcgcttg 3480

attgatcaaa aaccgatcag caatctcggt aatatccatg ccagcaataa catcatgttt 3540

aagttagacg gttgccgcga tacccaccgc tatatctgga tcaagtattt taacttattt 3600

gataaggaac tcaacgaaaa ggaaattaaa gacttatatg acaatcagag caatagcggc 3660

atcctgaagg atttctgggg cgactacctg cagtacgata agccgtacta tatgttgaac 3720

ttgtatgacc cgaacaaata tgtcgatgtg aacaatgtgg gtattcgtgg ctatatgtac 3780

ttaaagggcc cgcgtggtag cgtgatgacc acgaatattt acttaaacag cagcttatac 3840

cgcggcacga agtttattat caagaagtat gccagcggca acaaggacaa tatcgtccgc 3900

aacaacgacc gtgtgtatat taacgtggtg gtgaagaata aagagtaccg cttggccacg 3960

aatgcgagcc aggcgggcgt ggaaaaaatc ttgagcgcgt tggagatccc ggacgtcggc 4020

aacctcagcc aggttgtggt gatgaagtct aaaaacgacc agggcatcac gaacaagtgc 4080

aaaatgaatt tgcaagataa caacggcaac gacatcggct ttattggttt tcaccagttc 4140

aataacatcg ccaaactcgt ggccagcaat tggtataacc gccaaattga acgcagcagc 4200

cgcacgctcg gctgtagctg ggagttcatc ccggtggacg atggctgggg cgagcgcccg 4260

ctcggagatc tggtgccacg cggttccgcg aattcgagct ccgtcgacaa gctttggagc 4320

cacccgcagt tcgaaaaata a 4341

## Description

### FIELD OF THE INVENTION

This invention relates to novel recombinant clostridial neurotoxins exhibiting accelerated onset of effect and to methods for the manufacture of such recombinant clostridial neurotoxins. These novel recombinant clostridial neurotoxins comprise a domain consisting of proline, glycine and serine, and the methods comprise the steps of inserting a nucleic acid sequence coding for said domain into a nucleic acid sequence coding for a parental clostridial neurotoxin and expression of the recombinant nucleic acid sequence comprising the domain-coding sequence in a host cell. The invention further relates to novel recombinant single-chain precursor clostridial neurotoxins used in such methods, nucleic acid sequences encoding such recombinant single-chain precursor clostridial neurotoxins, and pharmaceutical compositions comprising the recombinant clostridial neurotoxin with accelerated onset of effect.

### BACKGROUND OF THE INVENTION

Clostridium is a genus of anaerobe gram-positive bacteria, belonging to the Firmicutes. Clostridium consists of around 100 species that include common free-living bacteria as well as important pathogens, such as *Clostridium botulinum* and *Clostridium tetani.* Both species produce neurotoxins, botulinum toxin and tetanus toxin, respectively. These neurotoxins are potent inhibitors of calcium-dependent neurotransmitter secretion of neuronal cells and are among the strongest toxins known to man. The lethal dose in humans lies between 0.1 ng and 1 ng per kilogram of body weight.

Oral ingestion of botulinum toxin via contaminated food or generation of botulinum toxin in wounds can cause botulism, which is characterised by paralysis of various muscles. Paralysis of the breathing muscles can cause death of the affected individual.

Although both botulinum neurotoxin (BoNT) and tetanus neurotoxin (TxNT) function via a similar initial physiological mechanism of action, inhibiting neurotransmitter release from the axon of the affected neuron into the synapse, they differ in their clinical response. While the botulinum toxin acts at the neuromuscular junction and other cholinergic synapses in the peripheral nervous system, inhibiting the release of the neurotransmitter acetylcholine and thereby causing flaccid paralysis, the tetanus toxin acts mainly in the central nervous system, preventing the release of the inhibitory neurotransmitters GABA (gamma-aminobutyric acid) and glycine by degrading the protein synaptobrevin. The consequent overactivity in the muscles results in generalized contractions of the agonist and antagonist musculature, termed a tetanic spasm (rigid paralysis).

While the tetanus neurotoxin exists in one immunologically distinct type, the botulinum neurotoxins are known to occur in seven different immunogenic types, termed BoNT/A through BoNT/H. Most *Clostridium botulinum* strains produce one type of neurotoxin, but strains producing multiple toxins have also been described.

Botulinum and tetanus neurotoxins have highly homologous amino acid sequences and show a similar domain structure. Their biologically active form comprises two peptide chains, a light chain of about 50 kDa and a heavy chain of about 100 kDa, linked by a disulfide bond. A linker or loop region, whose length varies among different clostridial toxins, is located between the two cysteine residues forming the disulfide bond. This loop region is proteolytically cleaved by an unknown clostridial endoprotease to obtain the biologically active toxin.

The molecular mechanism of intoxication by TeNT and BoNT appears to be similar as well: entry into the target neuron is mediated by binding of the C-terminal part of the heavy chain to a specific cell surface receptor; the toxin is then taken up by receptor-mediated endocytosis. The low pH in the so formed endosome then triggers a conformational change in the clostridial toxin which allows it to embed itself in the endosomal membrane and to translocate through the endosomal membrane into the cytoplasm, where the disulfide bond joining the heavy and the light chain is reduced. The light chain can then selectively cleave so called SNARE-proteins, which are essential for different steps of neurotransmitter release into the synaptic cleft, e.g. recognition, docking and fusion of neurotransmitter-containing vesicles with the plasma membrane. TeNT, BoNT/B, BoNT/D, BoNT/F, and BoNT/G cause proteolytic cleavage of synaptobrevin or VAMP (vesicle-associated membrane protein), BoNT/A and BoNT/E cleave the plasma membrane-associated protein SNAP-25, and BoNT/C cleaves the integral plasma membrane protein syntaxin and SNAP-25.

Clostridial neurotoxins display variable durations of action that are serotype specific. The clinical therapeutic effect of BoNT/A lasts approximately 3 months for neuromuscular disorders and 6 to 12 months for hyperhidrosis. The effect of BoNT/E, on the other hand, lasts less than 4 weeks. The longer lasting therapeutic effect of BoNT/A makes it preferable for certain clinical use compared to the other serotypes, for example serotypes B, C₁, D, E, F, G. One possible explanation for the divergent durations of action might be the distinct subcellular localizations of BoNT serotypes. The protease domain of BoNT/A light chain localizes in a punctate manner to the plasma membrane of neuronal cells, co-localizing with its substrate SNAP-25. In contrast, the short-duration BoNT/E serotype is cytoplasmic. Membrane association might protect BoNT/A from cytosolic degradation mechanisms allowing for prolonged persistence of BoNT/A in the neuronal cell.

The onset of the paralytic effect is also different in the neurotoxin serotypes. Whereas the onset of effect of BoNT/E in humans is observed after 0,5-1 day, the onset of the effect of BoNT/A in humans is only after 2-3 days. The peak effect is reached in humans after 1-2 days or 3-7 day after injections of BoNT/E or BoNT/A, respectively. Consequently BoNT/A has a late onset und a long duration of the paralytic effect, in contrast the paralytic effect of BoNT/E starts markedly earlier but lasts a markedly longer. The reason for this different onset and different duration of effect is not known.

In *Clostridium botulinum,* the botulinum toxin is formed as a protein complex comprising the neurotoxic component and non-toxic proteins. The accessory proteins embed the neurotoxic component thereby protecting it from degradation by digestive enzymes in the gastrointestinal tract. Thus, botulinum neurotoxins of most serotypes are orally toxic. Complexes with, for example, 450 kDa or with 900 kDa are obtainable from cultures of *Clostridium botulinum.*

In recent years, botulinum neurotoxins have been used as therapeutic agents in the treatment of dystonias and spasms. Preparations comprising botulinum toxin complexes are commercially available, e.g. from Ipsen Ltd (Dysport^{®}) or Allergan Inc. (Botox^{®}). A high purity neurotoxic component, free of any complexing proteins, is for example available from Merz Pharmaceuticals GmbH, Frankfurt (Xeomin^{®}).

Clostridial neurotoxins are usually injected into the affected muscle tissue, bringing the agent close to the neuromuscular end plate, i.e. close to the cellular receptor mediating its uptake into the nerve cell controlling said affected muscle. Various degrees of neurotoxin spread have been observed. The neurotoxin spread is thought to depend on the injected amount and the particular neurotoxin preparation. It can result in adverse side effects such as paralysis in nearby muscle tissue, which can largely be avoided by reducing the injected doses to the therapeutically relevant level. Overdosing can also trigger the immune system to generate neutralizing antibodies that inactivate the neurotoxin preventing it from relieving the involuntary muscle activity. Immunologic tolerance to botulinum toxin has been shown to correlate with cumulative doses.

At present, clostridial neurotoxins are still predominantly produced by fermentation processes using appropriate Clostridium strains. However, industrial production of clostridial neurotoxin from anaerobic Clostridium culture is a cumbersome and time-consuming process. Due to the high toxicity of the final product, the procedure must be performed under strict containment. During the fermentation process, the single-chain precursors are proteolytically cleaved by an unknown clostridial protease to obtain the biologically active di-chain clostridial neurotoxin. The degree of neurotoxin activation by proteolytic cleavage varies between different strains and neurotoxin serotypes, which is a major consideration for the manufacture due to the requirement of neurotoxin preparations with a well-defined biological activity. Furthermore, during fermentation processes using Clostridium strains the clostridial neurotoxins are produced as protein complexes, in which the neurotoxic component is embedded by accessory proteins. These accessory proteins have no beneficial effect on biological activity or onset of effect. They can however trigger an immune reaction in the patient, resulting in immunity against the clostridial neurotoxin. Manufacture of recombinant clostridial neurotoxins, which are not embedded by auxiliary proteins, might therefore be advantageous.

Methods for the recombinant expression of clostridial neurotoxins in *E. coli* are well known in the art (see, for example, WO 00/12728, WO 01/14570, or WO 2006/076902). Furthermore, clostridial neurotoxins have been expressed in eukaryotic expression systems, such as in *Pichia pastoris, Pichia methanolica, Saccharomyces cerevisiae,* insect cells and mammalian cells (see WO 2006/017749).

Recombinant clostridial neurotoxins may be expressed as single-chain precursors, which subsequently have to be proteolytically cleaved to obtain the final biologically active clostridial neurotoxin. Thus, clostridial neurotoxins may be expressed in high yield in rapidly-growing bacteria as relatively non-toxic single-chain polypeptides.

Furthermore, it might be advantageous to modify clostridial neurotoxin characteristics regarding biological activity, cell specificity, antigenic potential and onset of effect by genetic engineering to obtain recombinant neurotoxins with new therapeutic properties in specific clinical areas. Genetic modification of clostridial neurotoxins might allow altering the mode of action or expanding the range of therapeutic targets.

Botulinum toxin variant exhibiting a faster onset of effect in neuromuscular tissue than naturally occurring botulinum toxins would be advantageous for patients treated in aesthetic indications provided that the duration of effect is not reduced as it would be with BoNT/E. They would observe an almost immediate effect i.e. they could be treated just shortly before an important event to attain a prettier look. It is also advantageous for patients with neurological diseases (e.g. cervical dystonia, blepharospasm, spasticity). The patient would get a faster relief of the pain accompanying these diseases after the first injection or after the effect of the previous treatment has waned and the patient has to be injected again. Further new indications could be treated which require a fast paralysis of muscles. E.g. after surgery muscle could be blocked immediately so that the wound healing process is not inhibited by the movement of adjacent muscles.

There is a strong demand to produce new clostridial neurotoxins with a faster onset of effect with improved properties, in order to allow for exploitation of the therapeutic potential of BoNT serotypes. Ideally, the onset of effect of a particular clostridial neurotoxin could be adjusted in a tailor-made fashion in order to address any particular features and demands of a given indication, such as the amount of neurotoxin being administered, frequency of administration etc. In addition, it would be desirable to produce clostridial neurotoxins exhibiting a high specific activity. To date, such aspects have not been solved satisfactorily.

### OBJECTS OF THE INVENTION

It was an object of the invention to provide recombinant clostridial neurotoxins exhibiting a faster onset of effect in neuromuscular tissue than naturally occurring botulinum toxins. It was also an object of the invention to provide recombinant clostridial neurotoxins exhibiting a high specific activity. It was a further object of the invention to establish a reliable and accurate method for manufacturing and obtaining such recombinant clostridial neurotoxins. Such a method and novel precursor clostridial neurotoxins used in such methods would serve to satisfy the great need for recombinant clostridial neurotoxins exhibiting an accelerated onset of effect.

### SUMMARY OF THE INVENTION

The naturally occurring botulinum toxin serotypes display divergent onset of paralysis, possibly due to their distinct subcellular localization. BoNT/A exhibiting the slowest onset of effect was shown to localize in the vicinity of the plasma membrane of neuronal cells, whereas the fast acting BoNT/E serotype is cytosolic. However, additional factors such as degradation, diffusion, and/or translocation rates might have a decisive impact on the differences in the onset of effect for the individual botulinum toxin serotypes.

So far, no generally applicable method for modifying clostridial neurotoxins in order to accelerate the onset of effect is available. Surprisingly, it has been found that recombinant clostridial neurotoxins having such an effect can be obtained by cloning a sequence encoding a specific domain into a gene encoding a parental clostridial neurotoxin, and by subsequent heterologous expression of the generated construct in recombinant host cells, wherein this domain consists of proline, glycine and serine residues.

Thus, in one aspect, the present invention relates to recombinant clostridial neurotoxin comprising a domain, wherein this domain consists of proline, glycine and serine residues.

In another aspect, the present invention relates to a pharmaceutical composition comprising the recombinant clostridial neurotoxin of the present invention.

In yet another aspect, the present invention relates to the use of the composition of the present invention for cosmetic treatment.

In another aspect, the present invention relates to a method for the generation of the recombinant clostridial neurotoxin of the present invention, comprising the step of obtaining a recombinant nucleic acid sequence encoding a recombinant single-chain precursor clostridial neurotoxin by the insertion of a nucleic acid sequence encoding said domain into a nucleic acid sequence encoding a parental clostridial neurotoxin.

In another aspect, the present invention relates to a recombinant single-chain precursor clostridial neurotoxin comprising a domain consisting of proline, glycine and serine residues.

In another aspect, the present invention relates to a nucleic acid sequence encoding the recombinant single-chain precursor clostridial neurotoxin of the present invention.

In another aspect, the present invention relates to a method for obtaining the nucleic acid sequence of the present invention, comprising the step of inserting a nucleic acid sequence encoding said domain into a nucleic acid sequence encoding a parental clostridial neurotoxin.

In another aspect, the present invention relates to a vector comprising the nucleic acid sequence of the present invention, or the nucleic acid sequence obtainable by the method of the present invention.

In another aspect, the present invention relates to a recombinant host cell comprising the nucleic acid sequence of the present invention, the nucleic acid sequence obtainable by the method of the present invention, or the vector of the present invention.

In another aspect, the present invention relates to a method for producing the recombinant single-chain precursor clostridial neurotoxin of the present invention, comprising the step of expressing the nucleic acid sequence of the present invention, or the nucleic acid sequence obtainable by the method of the present invention, or the vector of the present invention in a recombinant host cell, or cultivating the recombinant host cell of the present invention under conditions that result in the expression of said nucleic acid sequence.

### FIGURES

**Figure 1****:** Schematic Presentation of **a** PGS-Botulinum Toxin A (PGS-100-rBoNT/A).
**Figure 2****:** SDS·PAGE of purified PGS-rBoNT/A. Prior to applying the samples to the gel, ß-mercaptoethanol was added.
   Lane "v.V." (before activation): purified, non-activated single-chain PGS-100-rBoNT/A having a molecular weight (Mw) of about 170 kDa.
   Lanes "n.A." (after activation) and "n.R." (after purification) show light chain (Lc-PGS-100-BoNT/A) and heavy chain (Hc) obtained after activation by thrombin under reducing conditions.

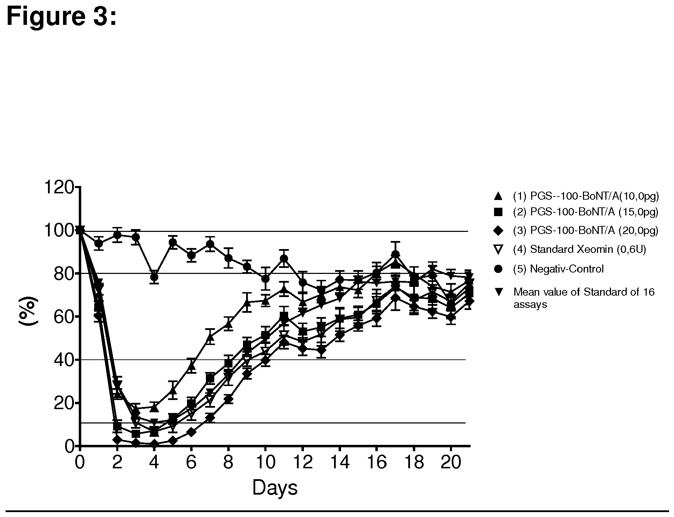

**Figure 3****:** Mouse running assay with PGS-100-rBoNT/A

### DETAILED DESCRIPTION OF THE INVENTION

The present invention may be understood more readily by reference to the following detailed description of the invention and the examples included therein.

In one aspect, the present invention relates to a recombinant clostridial neurotoxin comprising a domain consisting of proline, glycine and serine residues.

In the context of the present invention, the term "clostridial neurotoxin" refers to a natural neurotoxin obtainable from bacteria of the class Clostridia, including *Clostridium tetani* and *Clostridium botulinum,* or to a neurotoxin obtainable from alternative sources, including from recombinant technologies or from genetic or chemical modification. Particularly, the clostridial neurotoxins have endopeptidase activity.

Clostridial neurotoxins are produced as single-chain precursors that are proteolytically cleaved by an unknown clostridial endoprotease within the loop region to obtain the biologically active disulfide-linked di-chain form of the neurotoxin, which comprises two chain elements, a functionally active light chain and a functionally active heavy chain, where one end of the light chain is linked to one end of the heavy chain not via a peptide bond, but via a disulfide bond.

In the context of the present invention, the term "clostridial neurotoxin light chain" refers to that part of a clostridial neurotoxin that comprises an endopeptidase activity responsible for cleaving one or more proteins that is/are part of the so-called SNARE-complex involved in the process resulting in the release of neurotransmitter into the synaptic cleft: In naturally occurring clostridial neurotoxins, the light chain has a molecular weight of approx. 50 kDa.

In the context of the present invention, the term "clostridial neurotoxin heavy chain" refers to that part of a clostridial neurotoxin that is responsible for entry of the neurotoxin into the neuronal cell: In naturally occurring clostridial neurotoxins, the heavy chain has a molecular weight of approx. 100 kDa.

In the context of the present invention, the term "functionally active clostridial neurotoxin chain" refers to a recombinant clostridial neurotoxin chain able to perform the biological functions of a naturally occurring *Clostridium botulinum* neurotoxin chain to at least about 50%, particularly to at least about 60%, to at least about 70%, to at least about 80%, and most particularly to at least about 90%, where the biological functions of clostridial neurotoxin chains include, but are not limited to, binding of the heavy chain to the neuronal cell, entry of the neurotoxin into a neuronal cell, release of the light chain from the di-chain neurotoxin, and endopeptidase activity of the light chain. Methods for determining a neurotoxic activity can be found, for example, in WO 95/32738, which describes the reconstitution of separately obtained light and heavy chains of tetanus toxin and botulinum toxin. Also cell-based assay methods as described for example in WO2009/114748, WO 2013/049508 and WO2014/207109.

In the context of the present invention, the term "about" or "approximately" means within 20%, alternatively within 10%, including within 5% of a given value or range. Alternatively, especially in biological systems, the term "about" means within about a log (i.e. an order of magnitude), including within a factor of two of a given value.

In the context of the present invention, the term "recombinant clostridial neurotoxin" refers to a composition comprising a clostridial neurotoxin that is obtained by expression of the neurotoxin in a heterologous cell such as *E. coli,* and including, but not limited to, the raw material obtained from a fermentation process (supernatant, composition after cell lysis), a fraction comprising a clostridial neurotoxin obtained from separating the ingredients of such a raw material in a purification process, an isolated and essentially pure protein, and a formulation for pharmaceutical and/or aesthetic use comprising a clostridial neurotoxin and additionally pharmaceutically acceptable solvents and/or excipients.

In the context of the present invention, the term "recombinant clostridial neurotoxin" further refers to a clostridial neurotoxin based on a parental clostridial neurotoxin additionally comprising a heterologous domain wherein this domain consists of proline, glycine and serine residues, i.e. a domain that is not naturally occurring in said parental clostridial neurotoxin, in particular a synthetic domain, or a domain from a species other than *Clostridium botulinum,* in particular a domain from a human protein.

In the context of the present invention, the term "comprises" or "comprising" means "including, but not limited to". The term is intended to be open-ended, to specify the presence of any stated features, elements, integers, steps or components, but not to preclude the presence or addition of one or more other features, elements, integers, steps, components, or groups thereof. The term "comprising" thus includes the more restrictive terms "consisting of" and "consisting essentially of".

In particular embodiments, said domain consisting of proline, glycine and serine residues comprises an amino acid sequence consisting of at least 50 amino acid residues, particularly between 50 and 3000 amino acid residues, more particularly between 60 and 500 amino acid residues, more particularly between 70 and 260 amino acid residues, more particularly between 80 and 240 amino acid residues, more particularly between 90 and 220 amino acid residues, particularly 100 amino acid residues, 150 amino acid residues, or 200 amino acid residues.

Surprisingly, it has been found that the attachment of a domain consisting of proline, glycine and serine residues is able to expedite the onset of effect of a neurotoxin. A person skilled in the art would expect the opposite effect. It has been shown in WO2015/132004 that the attachment of a neurotoxin with Proline (P), Alanine (A) and Serine (S) residues delays the onset of effect. It has been speculated that the spread of a botulinum toxin might be reduced by an attachment of a domain consisting of certain amino acids due to a larger hydrodynamic radius of the fusion protein and thus, neurotoxin molecules could reach the neuromuscular junctions later inducing a delayed onset of effect. However, in the context of the present invention the attachment of a domain consisting of proline, glycine and serine residues surprisingly leads to a faster onset of effect.

In particular embodiments, said domain comprises a plurality of amino acid repeats, wherein said repeat consist of Pro, Gly, and Ser residues and wherein no more than six consecutive amino acid residues are identical.

In particular embodiments, the proline residues comprised in said domain constitute more than 4% and less than 40% of the amino acids of said domain.

In particular embodiments, said domain comprises at least the following amino acid sequence GSPGGPGPGSPGGPGPSGPG (SEQ ID NO: 1) or circular permuted versions or at least one multimer of the sequence as a whole or parts of the sequence, particularly (GSPGGPGPGSPGGPGPSGPG)ₙ, with n being an integer selected from 3 to 25, more particularly from 4 to 8, in particular wherein n is 5.

In particular embodiments, said domain is inserted at (i) the N-terminus of the light chain of said recombinant clostridial neurotoxin; (ii) the C-terminus of the light chain of said recombinant clostridial neurotoxin; (iii) the N-terminus of the heavy chain of said recombinant clostridial neurotoxin; or (iv) the C-terminus of the heavy chain of said recombinant clostridial neurotoxin; (v) in a loop of the tertiary structure of said recombinant clostridial neurotoxin.

In particular embodiments, the sequence of said clostridial neurotoxin is selected from the sequence of (i) a *Clostridium botulinum* neurotoxin serotype A, B, C, D, E, F, and G, particularly *Clostridium botulinum* neurotoxin serotype A, C and E, particularly *Clostridium botulinum* neurotoxin serotype A, or (ii) from the sequence of a functional variant of a *Clostridium botulinum* neurotoxin of (i), or (iii) from the sequence of a chimeric *Clostridium botulinum* neurotoxin, wherein the clostridial neurotoxin light chain and heavy chain are from different parental clostridial neurotoxin serotypes.

In the context of the present invention, the term *"Clostridium botulinum* neurotoxin serotype A, B, C, D, E, F, and G" refers to neurotoxins found in and obtainable from *Clostridium botulinum.* Currently, seven serologically distinct types, designated serotypes A, B, C, D, E, F, and G are known, including certain subtypes (e.g. A1, A2, A3, A4 and A5).

In particular embodiments the clostridial neurotoxin is selected from a *Clostridium botulinum* neurotoxin serotype A, B, C and E, in particular from *Clostridium botulinum* neurotoxin serotype A, or from a functional variant of any such *Clostridium botulinum* neurotoxin.

In particular embodiments, said recombinant clostridial neurotoxin has a light chain and a heavy chain comprised in the amino acid sequence as found in SEQ ID NO: 2.

In the context of the present invention, the term "functional variant of a clostridial neurotoxin" refers to a neurotoxin that differs in the amino acid sequence and/or the nucleic acid sequence encoding the amino acid sequence from a clostridial neurotoxin, but is still functionally active. In the context of the present invention, the term "functionally active" refers to the property of a recombinant clostridial neurotoxin to exhibit a biological activity of at least about 20%, particularly to at least about 50%, at least about 70%, at least about 80%, and most particularly at least about 90% of the biological activity of a naturally occurring parental clostridial neurotoxin, i.e. a parental clostridial neurotoxin without said domain, where the biological functions include, but are not limited to, binding to the neurotoxin receptor, entry of the neurotoxin into a neuronal cell, release of the light chain from the di-chain neurotoxin, and endopeptidase activity of the light chain, and thus inhibition of neurotransmitter release from the affected nerve cell. In vivo assays for assessing biological activity include the mouse LD50 assay and the ex vivo mouse hemidiaphragm assay as described by Pearce et al. (Pearce 1994, Toxicol. Appl. Pharmacol. 128: 69-77) and Dressler et al. (Dressler 2005, Mov. Disord. 20:1617-1619, Keller 2006, Neuroscience 139: 629-637) or a cell-based assay as described in WO2009/114748, WO2014/207109 or WO 2013/049508. The biological activity is commonly expressed in Mouse Units (MU). As used herein, 1 MU is the amount of neurotoxic component, which kills 50% of a specified mouse population after intraperitoneal injection, i.e. the mouse i.p. LD50.

On the protein level, a functional variant will maintain key features of the corresponding clostridial neurotoxin, such as key residues for the endopeptidase activity in the light chain, or key residues for the attachment to the neurotoxin receptors or for translocation through the endosomal membrane in the heavy chain, but may contain one or more mutations comprising a deletion of one or more amino acids of the corresponding clostridial neurotoxin, an addition of one or more amino acids of the corresponding clostridial neurotoxin, and/or a substitution of one or more amino acids of the corresponding clostridial neurotoxin. Particularly, said deleted, added and/or substituted amino acids are consecutive amino acids. According to the teaching of the present invention, any number of amino acids may be added, deleted, and/or substituted, as long as the functional variant remains biologically active. For example, 1, 2, 3, 4, 5, up to 10, up to 15, up to 25, up to 50, up to 100, up to 200, up to 400, up to 500 amino acids or even more amino acids may be added, deleted, and/or substituted. Accordingly, a functional variant of the neurotoxin may be a biologically active fragment of a naturally occurring neurotoxin. This neurotoxin fragment may contain an N-terminal, C-terminal, and/or one or more internal deletion(s).

In another embodiment, the functional variant of a clostridial neurotoxin additionally comprises a signal peptide. Usually, said signal peptide will be located at the N-terminus of the neurotoxin. Many such signal peptides are known in the art and are comprised by the present invention. In particular, the signal peptide results in transport of the neurotoxin across a biological membrane, such as the membrane of the endoplasmic reticulum, the Golgi membrane or the plasma membrane of a eukaryotic or prokaryotic cell. It has been found that signal peptides, when attached to the neurotoxin, will mediate secretion of the neurotoxin into the supernatant of the cells. In certain embodiments, the signal peptide will be cleaved off in the course of, or subsequent to, secretion, so that the secreted protein lacks the N-terminal signal peptide, is composed of separate light and heavy chains, which are covalently linked by disulfide bridges, and is proteolytically active.

In particular embodiments, the functional variant has in its clostridium neurotoxin part a sequence identity of at least about 40%, at least about 50%, at least about 60%, at least about 70% or most particularly at least about 80%, and a sequence homology of at least about 60%, at least about 70%, at least about 80%, at least about 90%, or most particularly at least about 95% to the corresponding part in the parental clostridial neurotoxin. Methods and algorithms for determining sequence identity and/or homology, including the comparison of variants having deletions, additions, and/or substitutions relative to a parental sequence, are well known to the practitioner of ordinary skill in the art. The term "identity" as used herein refers to sequence identity characterized by determining the number of identical amino acids between two nucleic acid sequences or two amino acid sequences wherein the sequences are aligned so that the highest order match is obtained. It can be calculated using published techniques or methods codified in computer programs such as, for example, BLASTP, BLASTN or FASTA (Altschul 1990, J Mol Biol 215, 403). The percent identity values are, in one aspect, calculated over the entire amino acid sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J Mol Biol 48; 443; Smith 1981, Adv Appl Math 2, 482), which are part of the GCG software packet (Genetics Computer Group 1991, 575 Science Drive, Madison, Wisconsin, USA 53711), may be used. The sequence identity values recited above in percent (%) are to be determined, in another aspect of the invention, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.On the DNA level, the nucleic acid sequences encoding the functional homologue and the parental clostridial neurotoxin may differ to a larger extent due to the degeneracy of the genetic code. It is known that the usage of codons is different between prokaryotic and eukaryotic organisms. Thus, when expressing a prokaryotic protein such as a clostridial neurotoxin, in a eukaryotic expression system, it may be necessary, or at least helpful, to adapt the nucleic acid sequence to the codon usage of the expression host cell, meaning that sequence identity or homology may be rather low on the nucleic acid level.

In the context of the present invention, the term "variant" refers to a neurotoxin that is a chemically, enzymatically, or genetically modified derivative of a corresponding clostridial neurotoxin, including chemically or genetically modified neurotoxin from *C. botulinum,* particularly of *C. botulinum* neurotoxin serotype A, C or E. A chemically modified derivative may be one that is modified by pyruvation, phosphorylation, sulfatation, lipidation, pegylation, glycosylation and/or the chemical addition of an amino acid or a polypeptide comprising between 2 and about 100 amino acids, including modification occurring in the eukaryotic host cell used for expressing the derivative. An enzymatically modified derivative is one that is modified by the activity of enzymes, such as endo- or exoproteolytic enzymes, including modification by enzymes of the eukaryotic host cell used for expressing the derivative. As pointed out above, a genetically modified derivative is one that has been modified by deletion or substitution of one or more amino acids contained in, or by addition of one or more amino acids (including polypeptides comprising between 2 and about 100 amino acids) to, the amino acid sequence of said clostridial neurotoxin. Methods for designing and constructing such chemically or genetically modified derivatives and for testing of such variants for functionality are well known to anyone of ordinary skill in the art.

In particular embodiments, said recombinant clostridial neurotoxin shows a faster onset of effect relative to an identical clostridial neurotoxin without a domain consisting of proline, glycine and serine residues.

In the context of the present invention, the term "accelerated onset of effect" or "accelerated onset of action" or "faster onset of action" or "faster onset of effect" refers to a faster or accelerated denervation mediated by a clostridial neurotoxin of the present invention. For example, as disclosed herein, administration of a disulfide-linked di-chain clostridial neurotoxin comprising a domain consisting of proline, glycine and serine residues results in localized paralysis for at an earlier time relative to administration of an identical disulfide-linked di-chain clostridial neurotoxin without the fusion of a polypeptide consisting of proline, glycine, and serine residues.

In the context of the present invention, the term "accelerated or faster onset of effect or action" is defined as a faster than about 20%, particularly faster than about 50%, more particularly faster than about 90% faster onset of effect of the recombinant neurotoxin of the present invention relative to the identical neurotoxin without the domain consisting of proline, glycine and serine residues. For example, a "faster onset of effect" can be determined using the "Mouse Running Assay". The "Mouse Running Assay" is well-known to the person skilled in the art and measures the daily running distance of a mouse in a treadmill after a botulinum neurotoxin was injected into the M. gastrocnemius (see Keller JE. Recovery from botulinum neurotoxin poisoning in vivo. Neuroscience. 2006 May 12;139(2):629-37). The distance which a mouse is able to run in the treadmill the day before the botulinum neurotoxin is injected is used as comparison and is set as 100%. A daily running distance of no more than 80% of the initial running distance is regarded as paralysis of the muscle. If the maximum paralytic effect is attained earlier than four days post injection of a botulinum neurotoxin serotype A, the botulinum neurotoxin serotype A is considered to exhibit a "faster onset of effect".

In the context of the present invention the term "maximum paralytic effect" refers to a value of about 90% reduction of the initial running distance. As disclosed herein, after administration of a recombinant clostridial neurotoxin according to the present invention maximum paralytic effect is attained 1 to 2 days earlier than after administration of an identical clostridial neurotoxin without a domain consisting of proline, glycine, and serine residues. Thus the present invention relates to novel recombinant clostridial neurotoxins exhibiting accelerated onset of effect.

In the context of the present invention the term "denervation" refers to denervation resulting from administration of a chemodenervating agent, for example a neurotoxin.

In the context of the present invention, the term "localized denervation" or "localized paralysis" refers to denervation of a particular anatomical region, usually a muscle or a group of anatomically and/or physiologically related muscles, which results from administration of a chemodenervating agent, for example a neurotoxin, to the particular anatomical region.

In the context of the present invention, the term "faster onset of effect" means that the domain consisting of proline, glycine and serine residues (PGS sequence) possibly enhances the degradation processes of SNAP25 in the cytosol of the neuron Thus, SNAP25, the substrate the proteolytic activity of the light chain in the neurons of injected muscles is earlier degraded by the light of the recombinant neurotoxin with the domain consisting of proline, glycine and serine residues relative to the light chain without the domain.

In particular embodiments of the invention, said recombinant clostridial neurotoxin comprising a domain consisting of proline, glycine and serine residues shows both i) a faster onset of effect relative to an identical clostridial neurotoxin without said domain and ii) a high specific activity.

In the context of the present invention, the term "high specific activity" means that the specific activity is > 25 Units / ng neurotoxin. The specific neurotoxin activity is defined as the biologic activity (in units) per mass of neurotoxin protein (in ng protein).

In another aspect, the present invention relates to a pharmaceutical or cosmetic composition comprising the recombinant clostridial neurotoxin of the present invention. For preparing a pharmaceutical preparation comprising a clostridial neurotoxin the toxin can be formulated by various techniques dependent on the desired application purposes which are known in the art. For example, the (biologically active) botulinum neurotoxin polypeptide can be used in combination with one or more pharmaceutically acceptable carriers as a pharmaceutical composition. The pharmaceutically acceptable carrier(s) must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatine, agar, pectin, acacia, magnesium stearate, stearic acid and the like. Exemplary of liquid carriers are glycerol, phosphate buffered saline solution, water, emulsions, various types of wetting agents, and the like. Suitable carriers comprise those mentioned above and others well known in the art, see, e.g., Remington's Pharmaceutical Sciences, Mack Publishing Company, Easton, Pennsylvania. In an aspect, the pharmaceutical composition can be dissolved in a diluent, prior to administration. The diluent is also selected so as not to affect the biological activity of the Neurotoxin product. Examples of such diluents are distilled water or physiological saline. In addition, the pharmaceutical composition or formulation may also include other carriers or non-toxic, non-therapeutic, non-immunogenic stabilizers and the like. Thus, the formulated Neurotoxin product can be present, in an aspect, in liquid or lyophilized form. In an aspect, it can be present together with glycerol, protein stabilizers (HSA) or non-protein stabilizers such as polyvinyl pyrrolidone (PVP), hyaluronic acid or free amino acids. In an aspect, suitable non-proteinaceous stabilizers are disclosed in WO 2005/007185 or WO 2006/020208. The formulated Neurotoxin product may be used for human or animal therapy of various diseases or disorders in a therapeutically effective dose or for cosmetic purposes.

In particular embodiments, the recombinant clostridial neurotoxin of the present invention or the pharmaceutical composition of the present invention is for use in the treatment of a disease or condition taken from the list of: wound healing, immobilisation for bone and tendon fracture treatment, post surgery immobilization, specifically in connection with haemorrhoidectomy, introduction of dental implants, or hip joint replacement (endoprothesis), epicondylitis, knee arthroplasty, ophthalmo logical surgery, acne, irritable bowel disease, vaginism, low back pain, or benign prostate dysplasia.

In yet another aspect, the present invention relates to the use of the composition of the present invention for cosmetic treatment.

In the context of the present invention, the term "cosmetic treatment" relates to uses in cosmetic or aesthetic applications, such as the treatment of wrinkles, crow's feet, glabella frown lines, reduction of the masseter muscle, reduction of the calves, removing of facial asymmetries etc..

In another aspect, the present invention relates to a method for the generation of the recombinant clostridial neurotoxin of the present invention, comprising the step of obtaining a recombinant nucleic acid sequence encoding a recombinant single-chain precursor clostridial neurotoxin by the insertion of a nucleic acid sequence encoding said domain consisting of proline, glycine and serine residues into a nucleic acid sequence encoding a parental clostridial neurotoxin.

In the context of the present invention, the term "recombinant nucleic acid sequence" refers to a nucleic acid, which has been generated by joining genetic material from two different sources.

In the context of the present invention, the term "single-chain precursor clostridial neurotoxin" refers to a single-chain precursor for a disulfide-linked di-chain clostridial neurotoxin, comprising a functionally active clostridial neurotoxin light chain, a functionally active neurotoxin heavy chain, and a loop region linking the C-terminus of the light chain with the N-terminus of the heavy chain.

In the context of the present invention, the term "recombinant single-chain precursor clostridial neurotoxin" refers to a single-chain precursor clostridial neurotoxin comprising a heterologous domain, i.e. a domain from a species other than *Clostridium botulinum.*

In particular embodiments, the recombinant single-chain precursor clostridial neurotoxin comprises a protease cleavage site in said loop region.

Single-chain precursor clostridial neurotoxins have to be proteolytically cleaved to obtain the final biologically active clostridial neurotoxins. Proteolytic cleavage may either occur during heterologous expression by host cell enzymes, or by adding proteolytic enzymes to the raw protein material isolated after heterologous expression. Naturally occurring clostridial neurotoxins usually contain one or more cleavage signals for proteases which post-translationally cleave the single-chain precursor molecule, so that the final di- or multimeric complex can form. At present, clostridial neurotoxins are still predominantly produced by fermentation processes using appropriate Clostridium strains. During the fermentation process, the single-chain precursors are proteolytically cleaved by an unknown clostridial protease to obtain the biologically active di-chain clostridial neurotoxin. In cases, where the single-chain precursor molecule is the precursor of a protease, autocatalytic cleavage may occur. Alternatively, the protease can be a separate non-clostridial enzyme expressed in the same cell. WO 2006/076902 describes the proteolytic cleavage of a recombinant clostridial neurotoxin single-chain precursor at a heterologous recognition and cleavage site by incubation of the *E. coli* host cell lysate. The proteolytic cleavage is carried out by an unknown *E. coli* protease. In certain applications of recombinant expression, modified protease cleavage sites have been introduced recombinantly into the interchain region between the light and heavy chain of clostridial toxins, e.g. protease cleavage sites for human thrombin or non-human proteases (see WO 01/14570).

In particular embodiments, the protease cleavage site is a site that is cleaved by a protease selected from the list of: a protease selected from the list of: thrombin, trypsin, enterokinase, factor Xa, plant papain, insect papain, crustacean papain, enterokinase, human rhinovirus 3C protease, human enterovirus 3C protease, tobacco etch virus protease, Tobacco Vein Mottling Virus, subtilisin and caspase 3.

In a particular embodiment, the recombinant single-chain precursor clostridial neurotoxin further comprises a binding tag, particularly selected from the group comprising: glutathione-S-transferase (GST), maltose binding protein (MBP), a Histag, a StrepTag, or a FLAG-tag.

In the context of the present invention, the term "parental clostridial neurotoxin" refers to an initial clostridial neurotoxin without a heterologous domain consisting of proline, glycine and serine residues, selected from a natural clostridial neurotoxin, a functional variant of a natural clostridial neurotoxin or a chimeric clostridial neurotoxin, wherein the clostridial neurotoxin light chain and heavy chain are from different clostridial neurotoxin serotypes.

In particular embodiments, the method for the generation of the recombinant clostridial neurotoxin of the present invention further comprises the step of heterologously expressing said recombinant nucleic acid sequence in a host cell, particularly in a bacterial host cell, more particularly in an *E. coli* host cell.

In certain embodiments, the *E. coli* cells are selected from *E. coli* XL1-Blue, Nova Blue, TOP10, XL10-Gold, BL21, and K12.

In particular embodiments, the method for the generation of the recombinant clostridial neurotoxin of the present invention additionally comprises at least one of the steps of (i) generating a disulfide-linked di-chain recombinant clostridial neurotoxin comprising said domain consisting of proline, glycine and serine residues by causing or allowing contacting of said recombinant single-chain precursor clostridial neurotoxin with an endoprotease and (ii) purification of said recombinant single-chain precursor clostridial neurotoxin or said disulfide-linked di-chain recombinant clostridial neurotoxin by chromatography.

In particular embodiments, the recombinant single-chain precursor clostridial neurotoxin, or the recombinant disulfide-linked di-chain clostridial neurotoxin, is purified after expression, or in the case of the recombinant disulfide-linked di-chain clostridial neurotoxin, after the cleavage reaction. In particular such embodiments, the protein is purified by chromatography, particularly by immunoaffinity chromatography, or by chromatography on an ion exchange matrix, a hydrophobic interaction matrix, or a multimodal chromatography matrix, particularly a strong ion exchange matrix, more particularly a strong cation exchange matrix.

In the context of the present invention, the term "causing ... contacting of said recombinant single-chain precursor clostridial neurotoxin ...with an endoprotease" refers to an active and/or direct step of bringing said neurotoxin and said endoprotease in contact, whereas the term "allowing contacting of a recombinant single-chain precursor clostridial neurotoxin ...with an endoprotease" refers to an indirect step of establishing conditions in such a way that said neurotoxin and said endoprotease are getting in contact to each other.

In the context of the present invention, the term "endoprotease" refers to a protease that breaks peptide bonds of non-terminal amino acids (i.e. within the polypeptide chain). As they do not attack terminal amino acids, endoproteases cannot break down peptides into monomers.

In particular embodiments, cleavage of the recombinant single-chain precursor clostridial neurotoxin is near-complete.

In the context of the present invention, the term "near-complete" is defined as more than about 95% cleavage, particularly more than about 97.5%, more particularly more than about 99% as determined by SDS-PAGE and subsequent Western Blot or reversed phase chromatography.

In particular embodiments, cleavage of the recombinant single-chain precursor clostridial neurotoxin occurs at a heterologous cleavage signal located in the loop region of the recombinant precursor clostridial neurotoxin.

In particular embodiments, the cleavage reaction is performed with crude host cell lysates containing said single-chain precursor protein.

In other particular embodiments, the single-chain precursor protein is purified or partially purified, particularly by a first chromatographic enrichment step, prior to the cleavage reaction.

In the context of the present invention, the term "purified" relates to more than about 90% purity. In the context of the present invention, the term "partially purified" relates to purity of less than about 90% and an enrichment of more than about two fold.

In another aspect, the present invention relates to a recombinant single-chain clostridial neurotoxin, which is a precursor for the recombinant clostridial neurotoxin of the present invention Thus, in such aspect, the present invention relates to a recombinant single-chain precursor clostridial neurotoxin comprising a domain consisting of proline, glycine and serine residues.

In particular embodiments, said domain consisting of proline, glycine and serine residues comprises an amino acid sequence consisting of at least 50 amino acid residues, particularly between 50 and 3000 amino acid residues, more particularly between 60 and 500 amino acid residues, more particularly between 70 and 260 amino acid residues, more particularly between 80 and 240 amino acid residues, more particularly between 90 and 220 amino acid residues, particularly 100 amino acid residues, 150 amino acid residues, or 200 amino acid residues.

In particular embodiments, said domain comprises a plurality of amino acid repeats, wherein said repeat consists of proline, glycine and serine residues and wherein no more than 6 consecutive amino acid residues are identical.

In particular embodiments, the proline residues comprised in said domain constitute more than 4% and less than 40% of the amino acids of said domain.

In particular embodiments, said domain comprises at least the following amino acid sequence GSPGGPGPGS PGGPGPSGPG (SEQ ID NO: 1) or a circular permuted version or at least one multimer of the sequence as a whole or parts of the sequence, particularly (GSPGGPGPGS PGGPGPSGPG)ₙ, with n being an integer selected from 3 to 25, in particular wherein n is 5.

In particular embodiments, said domain is inserted at (i) the N-terminus of the light chain of said recombinant clostridial neurotoxin; (ii) the C-terminus of the light chain of said recombinant clostridial neurotoxin; (i) the N-terminus of the heavy chain of said recombinant clostridial neurotoxin; or (ii) the C-terminus of the heavy chain of said recombinant clostridial neurotoxin; or a loop in the tertiary structure of the recombinant clostridial neurotoxin.

In a particular preferred embodiment of the invention the recombinant clostridial neurotoxin, comprises a domain consisting of (GSPGGPGPGS PGGPGPSGPG)₅ at the N-terminus of the light chain of said recombinant clostridial neurotoxin.

In particular embodiments, the sequence of said clostridial neurotoxin is selected from the sequence of (i) a *Clostridium botulinum* neurotoxin serotype A, B, C, D, E, F, and G, particularly *Clostridium botulinum* neurotoxin serotype A, C and E, more particularly *Clostridium botulinum* neurotoxin serotype A, or (ii) from the sequence of a functional variant of a *Clostridium botulinum* neurotoxin of (i), or (iii) from the sequence of a chimeric *Clostridium botulinum* neurotoxin, wherein the clostridial neurotoxin light chain and heavy chain are from different clostridial neurotoxin serotypes.

In particular embodiments of the present invention, said recombinant single-chain clostridial neurotoxin has the amino acid sequence as found in in SEQ ID NO: 2

In another aspect, the present invention relates to a nucleic acid sequence encoding the recombinant single-chain clostridial neurotoxin of the present invention, particularly a nucleic acid sequence as found in SEQ ID NO: 3

In another aspect, the present invention relates to a method for obtaining the nucleic acid sequence of the present invention, comprising the step of inserting a nucleic acid sequence encoding said domain into a nucleic acid sequence encoding a parental clostridial neurotoxin.

In another aspect, the present invention relates to a vector comprising the nucleic acid sequence of the present invention, or the nucleic acid sequence obtainable by the method of the present invention.

In another aspect, the present invention relates to a recombinant host cell comprising the nucleic acid sequence of the present invention, the nucleic acid sequence obtainable by the method of the present invention, or the vector of the present invention.

In certain embodiments, the recombinant host cells are selected from *E. coli* XL1 -Blue, Nova Blue, TOP10, XL10-Gold, BL21, and K12.

In another aspect, the present invention relates to a method for producing the recombinant single-chain precursor clostridial neurotoxin of the present invention, comprising the step of expressing the nucleic acid sequence of the present invention, or the nucleic acid sequence obtainable by the method of the present invention, or the vector of the present invention in a recombinant host cell, or cultivating the recombinant host cell of the present invention under conditions that result in the expression of said nucleic acid sequence.

### EXAMPLES

### Example 1: Generation and Purification of a PGS Botulinum Toxin Type A (PGS-100-rBoNT/A)

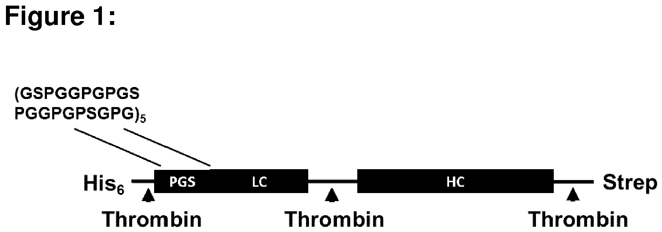

The "PGS" module comprising 100 amino acid residues built from the amino acids proline, glycine and serine was synthetically produced and after digestion with Ndel and Swal inserted at the N-terminus of recombinant BoNT/A (PGS-100-rBoNT/A) **(****Figure 1****).** The correct cloning was verified by sequencing.

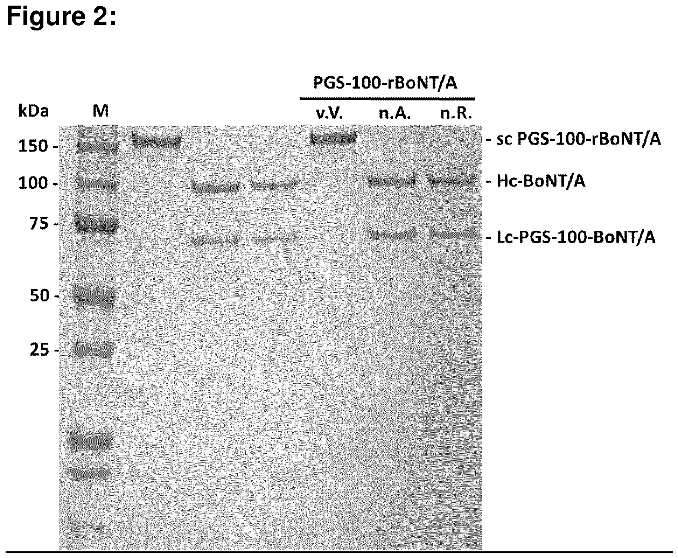

Expression was performed in expression strain *E. coli* BI21. Purification was done using a combination of his affinity, ion exchange and size exclusion chromatography, followed by activation using thrombin. **Figure 2** summarizes the results of purification and activation.

### Example 2: Onset of Effect of PGS-100-rBoNT/A in a "Mouse Running Assay"

Three different dosages of PGS-100-rBoNT/A, i.e. 10,0 pg, 15,0 pg and 20,0 pg were injected into the M. gastrocnemius of each mice in comparison to standard Xeomin^{®}. The mice had been trained in a treadmill. The daily running distance in the treadmill was measured over 20 days. The paralysis caused by the toxins was plotted as percentage of the running distance on the day before the injection, which was set as 100%, against the time (see **Figure 3**)**.**

The injection of PGS-100-rBoNT/A resulted in a maximum paralysis after 2-3 days, for the control group treated with Xeomin maximum paralysis was observed after 4 days. Thus, the onset of effective paralysis was markedly faster, whereas the recovery was not affected

### Table 1: Sequences

SEQ ID NO 1:
   GSPGGPGPGSPGGPGPSGPG
SEQ ID NO 2: PGS-100-BoNT/A 
MGSSHHHHHHGSLVPRSSSGSPGGPGPGSPGGPGPSGPGGSPGGPGPGSPGGPGPSGPGG
SPGGPGPGSPGGPGPSGPGGSPGGPGPGSPGGPGPSGPGGSPGGPGPGSPGGPGPSGPGG
PFVNKQFNYKDPVNGVDIAYIKIPNAGQMQPVKAFKIHNKIWVIPERDTFTNPEEGDLNP
PPEAKQVPVSYYDSTYLSTDNEKDNYLKGVTKLFERIYSTDLGRMLLTSIVRGIPFWGGS
TIDTELKVIDTNCINVIQPDGSYRSEELNLVIIGPSADIIQFECKSFGHEVLNLTRNGYG
STQYIRFSPDFTFGFEESLEVDTNPLLGAGKFATDPAVTLAHELIHAGHRLYGIAINPNR
VFKVNTNAYYEMSGLEVSFEELRTFGGHDAKFIDSLQENEFRLYYYNKFKDIASTLNKAK
SIVGTTASLQYMKNVFKEKYLLSEDTSGKFSVDKLKFDKLYKMLTEIYTEDNFVKFFKVL
NRKTYLNFDKAVFKINIVPKVNYTIYDGFNLRNTNLAANFNGQNTEINNMNFTKLKNFTG
LFEFYKLLCVRGIITSKAGAGKSLVPRGSAGAGALNDLCIKVNNWDLFFSPSEDNFTNDL
NKGEEITSDTNIEAAEENISLDLIQQYYLTFNFDNEPENISIENLSSDIIGQLELMPNIE
RFPNGKKYELDKYTMFHYLRAQEFEHGKSRIALTNSVNEALLNPSRVYTFFSSDYVKKVN
KATEAAMFLGWVEQLVYDFTDETSEVSTTDKIADITIIIPYIGPALNIGNMLYKDDFVGA
LIFSGAVILLEFIPEIAIPVLGTFALVSYIANKVLTVQTIDNALSKRNEKWDEVYKYIVT
NWLAKVNTQIDLIRKKMKEALENQAEATKAIINYQYNQYTEEEKNNINFNIDDLSSKLNE
SINKAMININKFLNQCSVSYLMNSMIPYGVKRLEDFDASLKDALLKYIYDNRGTLIGQVD
RLKDKVNNTLSTDIPFQLSKYVDNQRLLSTFTEYIKNIINTSILNLRYESNHLIDLSRYA
SKINIGSKVNFDPIDKNQIQLFNLESSKIEVILKNAIVYNSMYENFSTSFWIRIPKYFNS
 
ISLNNEYTIINCMENNSGWKVSLNYGEIIWTLQDTQEIKQRVVFKYSQMINISDYINRWI
FVTITNNRLNNSKIYINGRLIDQKPISNLGNIHASNNIMFKLDGCRDTHRYIWIKYFNLF
DKELNEKEIKDLYDNQSNSGILKDFWGDYLQYDKPYYMLNLYDPNKYVDVNNVGIRGYMY
LKGPRGSVMTTNIYLNSSLYRGTKFIIKKYASGNKDNIVRNNDRVYINVVVKNKEYRLAT
NASQAGVEKILSALEIPDVGNLSQVVVMKSKNDQGITNKCKMNLQDNNGNDIGFIGFHQF
NNIAKLVASNWYNRQIERSSRTLGCSWEFIPVDDGWGERPLGDLVPRGSANSSSVDKLWS
HPQFEK
SEQ ID NO 3: PGS-100-BoNT/A (nucleic acid sequence)

ATGGGTAGCAGCCATCATCATCACCATCATGGTAGCCTGGTTCCGCGTAGCAGCAGCGGT
AGTCCGGGTGGTCCGGGTCCGGGTTCACCTGGTGGTCCTGGACCGAGCGGTCCTGGTGGT
TCTCCAGGTGGCCCTGGTCCGGGAAGCCCAGGTGGACCAGGTCCTAGTGGTCCAGGCGGT
TCTCCTGGCGGTCCAGGTCCTGGATCTCCAGGCGGACCGGGTCCGTCAGGTCCAGGTGGT
AGCCCTGGTGGCCCAGGCCCTGGCTCACCGGGTGGACCTGGCCCTTCAGGACCGGGTGGG
TCACCTGGGGGACCGGGACCTGGAAGTCCTGGGGGTCCTGGTCCATCAGGCCCAGGCGGT
CCATTTGTGAACAAGCAGTTTAACTATAAGGACCCGGTGAACGGTGTGGATATCGCGTAT
ATCAAAATCCCGAATGCGGGCCAGATGCAACCAGTCAAGGCGTTCAAGATTCATAACAAG
ATTTGGGTTATTCCGGAACGTGATACCTTCACCAATCCGGAAGAAGGCGATTTAAATCCG
CCGCCAGAAGCCAAACAAGTGCCGGTGAGCTACTATGATAGCACGTATCTTAGCACCGAT
AATGAAAAAGACAATTACCTGAAGGGCGTGACCAAGTTGTTCGAGCGCATCTACAGTACC
GACTTAGGCCGCATGTTGTTGACGAGCATCGTTCGCGGTATCCCGTTCTGGGGCGGCTCG
ACCATTGATACCGAGTTGAAAGTCATTGACACGAACTGTATCAATGTTATCCAACCGGAC
GGCAGTTATCGCAGCGAGGAGTTAAATTTGGTCATCATCGGTCCAAGCGCAGATATTATT
CAGTTCGAATGCAAGAGCTTCGGCCATGAGGTCTTGAATTTGACGCGCAACGGTTACGGC
AGCACCCAATACATCCGCTTTAGCCCGGATTTCACCTTTGGCTTCGAGGAGAGCTTGGAG
GTGGACACCAACCCGCTGTTAGGTGCCGGCAAATTCGCAACCGACCCGGCAGTGACGTTG
GCGCACGAATTGATTCATGCGGGTCACCGCTTATACGGTATCGCGATCAATCCGAATCGC
GTCTTTAAAGTCAATACCAACGCGTACTACGAAATGAGCGGCTTAGAGGTTAGCTTTGAA
GAATTACGCACCTTCGGTGGCCACGACGCCAAGTTCATCGACAGCCTGCAGGAAAATGAG
TTCCGCTTGTACTATTACAATAAATTCAAGGACATCGCGAGCACCTTAAATAAAGCAAAG

AGCATTGTGGGCACCACCGCAAGCTTGCAGTACATGAAGAACGTATTTAAGGAAAAATAT
TTGTTGTCGGAGGATACCAGCGGGAAATTCAGCGTCGATAAGCTGAAATTCGACAAATTG
TATAAAATGCTGACCGAGATTTACACCGAGGATAACTTCGTCAAGTTTTTTAAGGTGTTA
AATCGTAAGACCTATTTAAACTTTGATAAAGCGGTGTTTAAAATTAATATCGTGCCGAAG
GTGAATTACACCATCTACGATGGTTTCAATTTACGCAACACGAATCTGGCGGCGAATTTT
AATGGCCAAAACACCGAAATTAACAACATGAACTTTACGAAGTTAAAGAATTTCACGGGC
TTATTCGAATTCTACAAGTTATTATGCGTGCGCGGCATCATTACCAGCAAGGCAGGTGCG
GGCAAGTCCTTGGTTCCGCGTGGCAGCGCCGGCGCCGGCGCGCTCAATGATCTGTGTATT
AAAGTCAATAACTGGGACCTGTTCTTCAGCCCGAGCGAGGATAACTTTACCAACGACTTA
AACAAAGGCGAGGAGATCACGAGCGATACGAACATCGAGGCGGCGGAGGAAAATATTAGC
CTGGACCTCATTCAGCAGTACTATCTGACGTTCAATTTTGACAATGAGCCGGAGAACATC
AGCATTGAAAATCTCAGCAGCGACATCATCGGTCAGTTGGAACTGATGCCGAACATTGAA
CGCTTTCCGAACGGCAAAAAATATGAACTGGACAAGTATACCATGTTCCATTACTTACGC
GCACAGGAATTTGAGCACGGCAAGAGCCGCATTGCGCTGACCAATAGCGTTAACGAGGCC
TTGTTAAATCCGAGCCGTGTCTACACGTTCTTCAGCAGCGATTATGTCAAAAAAGTGAAC
AAGGCGACCGAAGCCGCGATGTTTTTGGGCTGGGTCGAGCAATTGGTTTACGATTTTACC
GACGAAACCAGCGAGGTGAGCACGACCGACAAAATTGCAGATATCACCATCATCATTCCG
TACATCGGTCCGGCGCTCAATATCGGCAATATGTTATACAAGGACGACTTTGTGGGCGCG
CTGATCTTTAGCGGCGCGGTTATCTTATTAGAATTCATCCCGGAGATCGCAATCCCGGTC
TTGGGCACCTTTGCGTTGGTGAGCTATATCGCGAATAAAGTGCTCACGGTCCAAACCATC
GATAACGCGCTCAGCAAGCGTAATGAGAAATGGGACGAGGTTTATAAGTATATCGTGACC
AACTGGTTAGCAAAAGTCAATACGCAGATCGATCTCATCCGCAAAAAAATGAAAGAAGCC
TTGGAAAATCAAGCGGAGGCAACCAAAGCCATCATTAATTACCAGTATAACCAATATACC
GAAGAAGAAAAAAACAATATCAACTTCAATATCGATGATTTGAGCAGCAAACTGAACGAG
AGCATTAACAAAGCGATGATTAACATCAACAAGTTCTTGAATCAATGCAGCGTGAGCTAT
CTCATGAACAGCATGATCCCGTATGGCGTCAAACGCTTGGAAGATTTTGACGCCAGCCTG
AAAGATGCGCTCCTCAAGTATATTTATGACAACCGCGGCACCCTCATTGGCCAGGTGGAC
CGCTTGAAGGATAAAGTGAACAATACGCTCAGCACGGATATCCCGTTCCAGCTGAGCAAG
TACGTCGACAACCAGCGCTTACTGAGCACCTTTACCGAGTATATCAAGAACATCATTAAT
ACCAGCATCCTCAACTTGCGCTATGAGAGCAATCACCTGATCGACCTCAGCCGCTACGCC
AGCAAGATCAACATCGGCAGCAAGGTCAATTTCGACCCGATCGATAAGAATCAGATCCAA
TTGTTTAACCTGGAAAGCAGCAAGATCGAGGTTATCTTGAAGAACGCGATTGTGTACAAC
AGCATGTACGAGAACTTTAGCACGAGCTTCTGGATTCGTATCCCGAAGTATTTCAATAGC
ATTAGCCTGAATAACGAATATACCATTATCAACTGCATGGAAAATAATAGCGGCTGGAAG

GTGAGCTTAAATTACGGCGAGATCATTTGGACCTTACAGGATACCCAAGAAATCAAACAG
CGCGTCGTCTTTAAGTATAGCCAGATGATCAACATCAGCGATTACATCAACCGCTGGATC
TTCGTGACCATCACCAATAATCGCTTGAATAATAGCAAGATTTACATCAATGGTCGCTTG
ATTGATCAAAAACCGATCAGCAATCTCGGTAATATCCATGCCAGCAATAACATCATGTTT
AAGTTAGACGGTTGCCGCGATACCCACCGCTATATCTGGATCAAGTATTTTAACTTATTT
GATAAGGAACTCAACGAAAAGGAAATTAAAGACTTATATGACAATCAGAGCAATAGCGGC
ATCCTGAAGGATTTCTGGGGCGACTACCTGCAGTACGATAAGCCGTACTATATGTTGAAC
TTGTATGACCCGAACAAATATGTCGATGTGAACAATGTGGGTATTCGTGGCTATATGTAC
TTAAAGGGCCCGCGTGGTAGCGTGATGACCACGAATATTTACTTAAACAGCAGCTTATAC
CGCGGCACGAAGTTTATTATCAAGAAGTATGCCAGCGGCAACAAGGACAATATCGTCCGC
AACAACGACCGTGTGTATATTAACGTGGTGGTGAAGAATAAAGAGTACCGCTTGGCCACG
AATGCGAGCCAGGCGGGCGTGGAAAAAATCTTGAGCGCGTTGGAGATCCCGGACGTCGGC
AACCTCAGCCAGGTTGTGGTGATGAAGTCTAAAAACGACCAGGGCATCACGAACAAGTGC
AAAATGAATTTGCAAGATAACAACGGCAACGACATCGGCTTTATTGGTTTTCACCAGTTC
AATAACATCGCCAAACTCGTGGCCAGCAATTGGTATAACCGCCAAATTGAACGCAGCAGC
CGCACGCTCGGCTGTAGCTGGGAGTTCATCCCGGTGGACGATGGCTGGGGCGAGCGCCCG
CTCGGAGATCTGGTGCCACGCGGTTCCGCGAATTCGAGCTCCGTCGACAAGCTTTGGAGC
CACCCGCAGTTCGAAAAATAA

## Claims

1. A recombinant clostridial neurotoxin or a functional variant thereof comprising a domain wherein said domain comprises an amino acid sequence consisting of at least 50 amino acid residues, wherein said domain consists of proline, glycine and serine residues, or wherein said domain comprises a plurality of amino acid repeats, wherein said repeat consists of proline, glycine and serine residues.

2. The recombinant clostridial neurotoxin of claim 1, wherein no more than six consecutive amino acid residues are identical.

3. The recombinant clostridial neurotoxin of claim 1 or 2, wherein said domain comprises at least the following amino acid sequence (GSPGGPGPGS PGGPGPSGPG) (SEQ ID NO: 1) or circular permuted versions or multimers of the sequence as a whole or parts of the sequence, particularly (GSPGGPGPGS PGGPGPSGPG)ₙ, with n being an integer selected from 3 to 25, in particular wherein n is 5.

4. The recombinant clostridial neurotoxin of claim 1 to 3, wherein said domain is inserted at (i) the N-terminus of the light chain of said recombinant clostridial neurotoxin; (ii) the C-terminus of the light chain of said recombinant clostridial neurotoxin; (iii) the N-terminus of the heavy chain of said recombinant clostridial neurotoxin; or (iv) the C-terminus of the heavy chain of said recombinant clostridial neurotoxin.

5. The recombinant clostridial neurotoxin of any one of claims 1 to 4, wherein the sequence of said clostridial neurotoxin is selected from the sequence of (i) a *Clostridium botulinum* neurotoxin serotype A, B, C, D, E, F, and G, or (ii) from the sequence of a functional variant of a *Clostridium botulinum* neurotoxin of (i), or (iii) from the sequence of a chimeric *Clostridium botulinum* neurotoxin, wherein the clostridial neurotoxin light chain and heavy chain are from different clostridial neurotoxin serotypes.

6. The recombinant clostridial neurotoxin of claim 5, wherein said recombinant clostridial neurotoxin has a light chain and a heavy chain comprised in the amino acid sequence as found in SEQ ID NO: 2.

7. The recombinant clostridial neurotoxin of any one of claims 1 to 6, wherein said recombinant clostridial neurotoxin shows an accelerated onset of effect relative to an identical clostridial neurotoxin without said domain.

8. A pharmaceutical composition comprising the recombinant clostridial neurotoxin of any one of claims 1 to 7.

9. Use of the recombinant clostridial neurotoxin of any one of claims 1 to 7 for cosmetic treatment.

10. A method for the generation of a recombinant clostridial neurotoxin according to any one of claims 1 to 7, comprising the step of obtaining a recombinant nucleic acid sequence encoding a recombinant single-chain precursor clostridial neurotoxin by the insertion of a nucleic acid sequence encoding said domain into a nucleic acid sequence encoding a parental clostridial neurotoxin, or wherein said method further comprises the step of heterologously expressing said recombinant nucleic acid sequence in a host cell, particularly in a bacterial host cell, more particularly in an *E. coli* host cell.

11. A recombinant single-chain clostridial neurotoxin, which is a precursor for the recombinant clostridial neurotoxin of any one of claims 1 to 7, e. g. wherein the sequence of said clostridial neurotoxin is selected from the sequence of (i) a Clostridium botulinum neurotoxin serotype A, B, C, D, E, F, and G, or (ii) from the sequence of a functional variant of a Clostridium botulinum neurotoxin of (i), or (iii) from the sequence of a chimeric Clostridium botulinum neurotoxin, wherein the clostridial neurotoxin light chain and heavy chain are from different clostridial neurotoxin serotypes.

12. A nucleic acid sequence encoding the recombinant single-chain clostridial neurotoxin of claim 11, particularly a nucleic acid sequence as found in SEQ ID NO: 3.

13. A method for obtaining the nucleic acid sequence of claim 12, comprising the step of inserting a nucleic acid sequence encoding said domain into a nucleic acid sequence encoding a parental clostridial neurotoxin.

14. A vector comprising the nucleic acid sequence of claim 12, or the nucleic acid sequence obtainable by the method of claim 13.

15. A recombinant host cell comprising the nucleic acid sequence of claim 12, the nucleic acid sequence obtainable by the method of claim 13, or the vector of claim 14.
